# EUROPEAN PATENT SPECIFICATION

(11) **EP 2 962 685 B1**
(45) Date of publication and mention of the grant of the patent: **08.01.2020**
(21) Application number: 15174276.4
(22) Date of filing: 29.06.2015
(51) Int. Cl.: A61K 9/20, A61K 31/4985

(54) **ORALLY DISINTEGRATING FORMULATIONS OF TADALAFIL**
IM MUND ZERFALLENDE FORMULIERUNGEN VON TADALAFIL
FORMULATIONS DE TADALAFIL SE DÉSINTÉGRANT PAR VOIE ORALE

(30) Priority: 30.06.2014 TR 201407627; 25.06.2015 TR 201507833
(43) Date of publication of application: 06.01.2016
(73) Proprietor: MONTERO GIDA SANAYI VE TICARET A.S., Istinye, Istanbul 34460 (TR)
(72) Inventor: TÜRKYILMAZ, Ali, 34460 Istanbul (TR); YELKEN, Gülay, 34460 Istanbul (TR); GÜNER, Dicle, 34460 Istanbul (TR); ZAN, Merve, 34460 Istanbul (TR)
(74) Representative: Sevinç, Erkan

(56) References cited:
- EP-A1- 2 698 145
- WO-A2-2006/047493
- CN-A- 103 271 885

## Description

### Field of Invention

The present invention relates to an orally disintegrating pharmaceutical formulation comprising tadalafil or a pharmaceutically acceptable salt thereof and crospovidon.

### The background of the invention

Tadalafil is an orally administered phosphodiesterase type 5 (PDE5) inhibitor that has been developed as a treatment for erectile dysfunction and pulmonary arterial hypertension. It has a longer half life compared to other PDE5 inhibitors (mean 17,5 hours). Its chemical name is (6R-trans)-6-(1,3-benzodioxol-5-yl)- 2,3,6,7,12,12a-hexahydro-2-methyl-pyrazino [1', 2':1,6] pyrido[3,4-b]indole-1,4-dione. Its chemical structure is shown in Formula I. It is freely soluble only in solvents such as dimethylsulfoxide and dimethylformamide and not soluble in water.

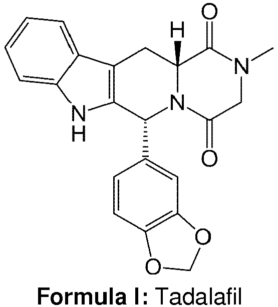

Tadalafil is first disclosed in US5859006A. In US718295 8B1, tablet formulations of tadalafil are disclosed. Moreover, film coated tablets of tadalafil are also available in the market in the strength of 2.5mg, 5mg, 10mg, and 20mg.

Over the past decades, orally disintegrating tablets (ODTs) have gained considerable attention as a preferred alternative to conventional tablets and capsules due to better patient compliance. ODTs are solid dosage forms containing active ingredients which disintegrate rapidly through buccal mucosa. It is desirable in the treatment of a number of diseases. They are also advantageous for administrations of medicaments to patients who are traveling or have little access to water are similarly affected or patients who have difficulties swallowing other dosage forms.

There are various patent applications in terms of orally disintegrating or mouth dissolving agents.

EP 2 698 145 A1 discloses formulations containing a combination of dapoxetine or a pharmaceutically acceptable salt thereof with a phosphodiesterase Type 5 inhibitor or a pharmaceutically acceptable salt thereof. A process for preparing a similar formulation and its use in treatment of erectile dysfunction is also disclosed.

CN 10 3271 885 A discloses a tadalafil orally disintegrating tablet and a preparation method thereof. The tadalafil orally disintegrating tablet contains pharmaceutically acceptable auxiliary materials such as filling agents, disintegrating agents, flavouring agents and lubricating agents, and when being taken, dispenses with water, can be disintegrated or dissolved in oral cavities.

WO 2006/047493 A2 discloses a method for preparing an orally disintegrating tablet (ODT) composition comprising microparticles of one or more taste-masked active pharmaceutical ingredient(s), rapidly-dispersing microgranules, and other optional, pharmaceutically acceptable excipients wherein the ODT disintegrates on contact with saliva in the buccal cavity forming a smooth, easy-to-swallow suspension.

However, there are already some challenges about ODT formulations of tadalafil in terms of stability and solubility. In prior art, in order to solve low solubility problem of tadalafil lactose has been used as a diluent. However, lactose can cause instability problems due to moisture sensitivity of orally disintegrating tablets.

In this invention, to overcome stability and solubility problems of tadalafil, mannitol has been used. Moreover, to improve the flowability of formulation, mannitol and crospovidon are used in specific particle sizes.

### Detailed description of the invention

The present invention provides an orally disintegrating pharmaceutical formulation comprising tadalafil or a pharmaceutically acceptable salt thereof and crospovidon wherein the value of the particle diameter at 50% in the cumulative distribution (d50) of crospovidon is between 3 to 50 µm, measured by Malvern Mastersizer 2000 laser diffraction particle size analyser.

The main embodiment of this present invention is to obtain rapidly disintegrating and stable formulations that comprise tadalafil.

According to one embodiment of this present invention is to provide an orally disintegrating formulation wherein tadalafil or a pharmaceutically acceptable salt thereof is present in an amount of 1 to 80 % by weight of total formulation, preferably 1 to 60 %, more preferably it is 1 to 40 % by weight of total formulation.

The orally disintegrating formulation of this present invention comprises at least one pharmaceutically acceptable excipient which is selected from the group comprising diluents, super disintegrants, lubricants, taste masking coating agents, binders, glidants, sweeteners, flavouring agents, acidifying agents and alkalizing agents.

In one embodiment, the diluent is selected from the group comprising mannitol, dicalcium phosphate, calcium carbonate, calcium sulphate dehydrate, sugars, sorbitol, sucrose, inorganic salts, calcium salts, polysaccharides, dextrose, sodium chloride, dextrates, lactitol, maltodextrin, sucrose-maltodextrin mixtures, xylitol, trehalose, heavy magnesium carbonate, gallen IQ (isomalt), microcrystalline cellulose, lactose, starch, sodium carbonate, sodium bicarbonate, LudiFlash (mannitol, crospovidon and polyvinyl acetate), Pharmaburst, Panexcea (microcrystalline cellulose, HPMC and crospovidon), F-Melt and mixtures thereof, preferably it is mannitol.

It is difficult to develop orally disintegrating formulations because of several different reasons and requirements such as rapid disintegration, stability and solubility. Dosage form must disintegrate in the oral cavity with the existence of saliva in a short period of time. So those formulations should have a porous structure. However, these porous characteristic tend to be very sensitive to humidity and may be lead to instability problems. ODTs have porous structure and it makes them more sensitive to moisture during shelf life as compared to conventional tablets. In this present invention, to achieve stability of the orally disintegrating tablets of tadalafil, a non-hygroscopic diluent is selected.

According to one embodiment, diluent used in this present invention is non-hygroscopic diluent. The non-hygroscopic diluent is selected from the group comprising mannitol, dicalcium phosphate, calcium carbonate, calcium sulphate dihydrate and mixtures thereof.

One embodiment of the present invention is to provide a formulation comprising mannitol. Mannitol is used as a diluent that also provides the stability of formulation. Mannitol is one of the most suitable diluents for moisture-sensitive formulations, coupled with a pleasant taste and mouthfeel. It is inert, non-hygroscopic diluent used in formulations that are moisture sensitive. In prior art, for the tablet formulations of tadalafil, lactose is mostly used. The reason to prefer lactose instead of mannitol is solubility problem of tadalafil. However, using lactose in orally disintegrating tablets of tadalafil is not suitable due to hygroscopic structure of the formulations. ODTs have porous structure and it makes them more sensitive to moisture during shelf life as compared to conventional tablets. In this present invention, to achieve stability of the orally disintegrating tablets of tadalafil, mannitol is used as a diluent, while surprisingly achieving solubility problem of tadalafil.

In one embodiment, in the formulation of this present invention, non-hygroscopic diluent is mannitol. To achieve solubility of tadalafil, specific particle size of mannitol has been used. In that way, it has been surprisingly found that both stability and solubility problem of tadalafil in orally disintegrating tablet has been overcome.

According to this embodiment, mannitol has a particle size in diameter range with a d₅₀ value. The particle size of mannitol in diameter range with a d₅₀ value is between 10 to 200 µm, preferably 20 to 100 µm and more preferably it is 30 to 60 µm.

According to one embodiment, in addition to crospovidon, the super-disintegrant is selected from the group comprising povidone, alginic acid and alginates, ion exchange resins, magnesium aluminum silica, sodium dodecyl sulphate, croscarmellose sodium, sodium carboxymethyl starch, carboxy methyl cellulose calcium, docusate sodium, sodium starch glycolate, sodium glysin carbonate, sodium lauryl sulphate, soy polysaccharide, low substituted HPC(hydroxy propyl cellulose), polyacrylin potasium, poloxamer, guar gum, gellan gum, xanthan gum, calcium silicate and mixtures thereof.

The super disintegrant is crospovidon in the formulation of this present invention to provide porous ODT structure. It has been used in small particle size to further enhance the solubility of tadalafil. The particle size of crospovidon in diameter range with a d₅₀ value is between 3 to 50 µm, preferably 5 to 30 µm and more preferably it is 7 to 20 µm. With these particle sizes of crospovidone, it has been surprisingly found that solubility of the formulation has been increased.

In this invention, particle sizes of mannitol and crospovidon have been measured by *Malvern Mastersizer 2000 laser diffraction particle size analyzer* with solid samples and d₅₀ value of these excipients have been identified. "d₅₀" or "d(0.5)" is defined as the median diameter or the medium value of the particle size distribution, it is the value of the particle diameter at 50% in the cumulative distribution.

The lubricants are selected from the group comprising sodium stearyl fumarate, magnesium strearate, calcium stearate, sodium lauryl sulphate, magnesium lauryl sulphate, stearic acid, polyethylene glycol, paraffin, fumaric acid, glyceril palmitostearate, hydrogenated vegetable oil, zinc stearate and mixtures thereof. The lubricant used in this present invention is sodium stearyl fumarate.

On the other side, using small size of mannitol and crospovidon affects the flowability in a bad way and results in compressibility problems. To overcome these problems, the ratio of crospovidon to sodium stearyl fumarate is used in a specific ratio. It has been surprisingly found that with this ratio, desired flowability has been achieved to compress the formulation into ODTs.

According to this embodiment, the ratio of crospovidon to sodium stearyl fumarate is in the range of 1 to 100 (w/w), preferably 3 to 25 (w/w) and more preferably 5 to 20 (w/w).

Suitable binders are selected from the group comprising polyvinylpyrrolidone (povidone), sugars, glycose syrup, natural gums, gelatin, collagene, proteins such as gelatin, agar, alginates, carbomers, carboxymethylcellulose sodium, cellulose acetate phthalate, chitosan, copovidone, starch, corn starch and pregelatinized starch, starch mucilage, acacia mucilage, dextrates,dextrin, dextrose, ethylcellulose, glyceryl behenate, guar gum, hydrogenated vegetable oil type I, hydroxyethyl cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, aluminia hydroxide, stearic acid, sucrose, bentonite, laponit, setostearyl alcohol, polyoxyethilene-alkyl ethers, pullulan or mixtures thereof.

Suitable sweeteners are selected from the group comprising sucralose, aspartame, saccharin, sodium cyclamate, acesulfam-K, sugars such as glucose, lactose, fructose and sugar alcohols such as mannitol, sorbitol, xylitol, erythritol or mixtures thereof; preferably sucralose.

Suitable flavouring agents are selected from the group comprising menthol, peppermint, cinnamon, chocolate, vanillin and fruit essences such as cherry, orange, strawberry, grape or mixtures thereof.

Suitable glidants are selected from the group comprising colloidal silicon dioxide, colloidal anhydrous silica, talc, aluminium silicate or mixtures thereof.

Suitable taste-masking coating agents are selected from the group comprising aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene glycol, polyethylene oxide, poly-DL-(lactic acid), polyvinyl acetate phthalate, polyvinyl alcohol, potassium chloride, povidone, shellac, shellac with stearic acid, sucrose, surface color agents, titanium oxide, tributyl citrate, triethyl citrate, vanillin, white wax, xylitol, yellow wax, zein, dimethylaminoethyl methacrylate, butyl methacrylate and methyl methacrylate (Eudragit E 100) (Poly(butyl methacrylate-co-(2- demethylaminoeethyl)methacrylate-co-methyl methacrylate)) or mixture of polyethylene glycol and polyvinyl alcohol (Kollicoat IR) and their copolymers and their mixtures.

Suitable acidifying agents may include but not limited to citric acid, fumaric acid, adipic acid, acetic acid, hydrochloric acid, malic acid, nitric acid, phosphoric acid, sulfuric acid, tartaric acid and mixtures thereof.

Suitable alkalizing agents may include but not limited to sodium bicarbonate, sodium glycine carbonate, ammonia solution, ammonium carbonate, diethanolamine, diisopropanolamine, potassium hydroxide, sodium carbonate, sodium borate, sodium hydroxide, trolamine and mixtures thereof.

| **Ingredients** | **% (amount)** |
|---|---|
| tadalafil | 1.0 - 80.0 % |
| mannitol 60 | 5.0 - 90.0 % |
| mannitol DC 400 | 5.0 - 90.0 % |
| polyvinylpyrrolidone K-30 | 0.1 - 25.0 % |
| crospovidon CL-SF | 5.0 - 60.0 % |
| sodium lauryl sulphate | 0.01 - 10.0 % |
| sucralose | 0.01 - 3.0 % |
| flavouring agent | 0.1 - 5.0 % |
| dyeing agent | 0.001 - 3.0 % |
| sodium stearyl fumarate | 0.1 - 10.0 % |
| water | q.s. |

The production of the formulation is carried out as follows: granulation solution is prepared by mixing of sodium lauryl sulphate, polyvinylpyrrolidone (K-30) and dyeing agent with water. Mannitol 60, half of crospovidon CL-SF and tadalafil are sieved and mixed. Then, granulation is performed with granulation solution. Wet granules are seieved and dried. Dried granules are sieved and mixed with other half of crospovidon CL-SF, sucralose, flavouring agent and mannitol DC400. Then, sodium stearyl fumarate is sieved and added to mixture and mixed again. Final powder mixture is pressed into tablets.

## Claims

1. An orally disintegrating pharmaceutical formulation comprising tadalafil or a pharmaceutically acceptable salt thereof and crospovidon wherein the value of the particle diameter at 50% in the cumulative distribution (d50) of crospovidon is between 3 to 50 µm, measured by Malvern Mastersizer 2000 laser diffraction particle size analyzer.

2. The orally disintegrating pharmaceutical formulation according to claim 1, further comprises at least one pharmaceutically acceptable excipient selected from the group comprising diluents, super disintegrants, lubricants, taste masking coating agents, binders, glidants, sweeteners, flavouring agents, acidifying agents and alkalizing agents.

3. The orally disintegrating pharmaceutical formulation according to claim 2, wherein diluent is non-hygroscopic diluent.

4. The orally disintegrating pharmaceutical formulation according to claim 3, wherein the non-hygroscopic diluent is selected from the group comprising mannitol, dicalcium phosphate, calcium carbonate, calcium sulphate dihydrate and mixtures thereof.

5. The orally disintegrating pharmaceutical formulation according to claim 4, wherein the non-hygroscopic diluent is mannitol.

6. The orally disintegrating pharmaceutical formulation according to claim 5, wherein the value of the particle diameter at 50% in the cumulative distribution (d50) of mannitol is between 10 to 200 µm, preferably 20 to 100 µm and more preferably it is 30 to 60 µm, measured by Malvern Mastersizer 2000 laser diffraction particle size analyzer.

7. The orally disintegrating pharmaceutical formulation according to claim 1, wherein the value of the particle diameter at 50% in the cumulative distribution (d50) of crospovidon is between 5 to 30 µm, preferably 7 to 20 µm, measured by Malvern Mastersizer 2000 laser diffraction particle size analyzer..

8. The orally disintegrating pharmaceutical formulation according to claim 2, wherein lubricant is sodium stearyl fumarate.

9. The orally disintegrating pharmaceutical formulation according to any preceeding claim, wherein the ratio of crospovidon to sodium stearyl fumarate is in the range of 1 to 100 (w/w), preferably 3 to 25 (w/w) and more preferably 5 to 20 (w/w).

## Patentansprüche

1. Orodispersible pharmazeutische Formulierung enthaltend Tadalafil oder ein pharmazeutisch verträgliches Salz davon und Crospovidon, wobei der Wert des Partikeldurchmessers bei 50 % in der kumulativen Verteilung (d50) von Crospovidon zwischen 3 bis 50 µm liegt, gemessen mit einem Analysegerät für die Bestimmung der Partikelgröße anhand von Laserbeugung vom Typ Malvern Mastersizer 2000.

2. Orodispersible pharmazeutische Formulierung nach Anspruch 1, welche weiterhin wenigstens einen pharmazeutisch verträglichen Hilfsstoff enthält, der aus der Gruppe ausgewählt ist, welche Verdünnungsmittel, Super-Sprengmittel, Schmiermittel, geschmacksmaskierende Beschichtungsmittel, Bindemittel, Gleitmittel, Süßstoffe, Geschmacksstoffe, sowie Mittel zum Ansäuern und Mittel zum Alkalisieren umfasst.

3. Orodispersible pharmazeutische Formulierung nach Anspruch 2, wobei das Verdünnungsmittel ein Verdünnungsmittel ist, das nicht hygroskopisch ist.

4. Orodispersible pharmazeutische Formulierung nach Anspruch 3, wobei das Verdünnungsmittel, das nicht hygroskopisch ist, aus der Gruppe ausgewählt ist, die Mannitol, Dicalciumphosphat, Calciumcarbonat, Calciumsulfatdihydrat und Gemische davon umfasst.

5. Orodispersible pharmazeutische Formulierung nach Anspruch 4, wobei das Verdünnungsmittel, das nicht hygroskopisch ist, Mannitol ist.

6. Orodispersible pharmazeutische Formulierung nach Anspruch 5, wobei der Wert des Partikeldurchmessers bei 50 % in der kumulativen Verteilung (d50) von Mannitol zwischen 10 bis 200 µm, vorzugsweise 20 bis 100 µm und noch bevorzugter 30 bis 60 µm, liegt, gemessen mit einem Analysegerät für die Bestimmung der Partikelgröße anhand von Laserbeugung vom Typ Malvern Mastersizer 2000.

7. Orodispersible pharmazeutische Formulierung nach Anspruch 1, wobei der Wert des Partikeldurchmessers bei 50 % in der kumulativen Verteilung (d50) von Crospovidon zwischen 5 bis 30 µm, vorzugsweise 7 bis 20 µm, liegt, gemessen mit einem Analysegerät für die Bestimmung der Partikelgröße anhand von Laserbeugung vom Typ Malvern Mastersizer 2000.

8. Orodispersible pharmazeutische Formulierung nach Anspruch 2, wobei das Schmiermittel Natriumstearylfumarat ist.

9. Orodispersible pharmazeutische Formulierung nach einem der vorangehenden Ansprüche, wobei das Verhältnis von Crospovidon zu Natriumstearylfumarat in dem Bereich von 1 bis 100 (m/m), vorzugsweise 3 bis 25 (m/m) und noch bevorzugter 5 bis 20 (m/m) liegt.

## Revendications

1. Formulation pharmaceutique se désintégrant par voie orale, comprenant du tadalafil ou un sel pharmaceutiquement acceptable de celui-ci et de la crospovidone, dans laquelle la valeur du diamètre de particule à 50 % dans la distribution cumulée (d50) de crospovidone est entre 3 et 50 µm, mesurée par un analyseur de dimension de particule à diffraction laser Mastersizer 2000 de Malvern.

2. Formulation pharmaceutique se désintégrant par voie orale selon la revendication 1, comprenant en outre au moins un excipient pharmaceutiquement acceptable choisi dans le groupe comprenant les diluants, les super désintégrants, les lubrifiants, les agents d'enrobage masquant le goût, les liants, les agents de glissement, les édulcorants, les agents aromatisants, les agents acidifiants et les agents alcalinisants.

3. Formulation pharmaceutique se désintégrant par voie orale selon la revendication 2, dans laquelle un diluant est un diluant non hygroscopique.

4. Formulation pharmaceutique se désintégrant par voie orale selon la revendication 3, dans laquelle le diluant non hygroscopique est choisi dans le groupe comprenant le mannitol, le phosphate dicalcique, le carbonate de calcium, le sulfate de calcium dihydraté et leurs mélanges.

5. Formulation pharmaceutique se désintégrant par voie orale selon la revendication 4, dans laquelle le diluant non hygroscopique est le mannitol.

6. Formulation pharmaceutique se désintégrant par voie orale selon la revendication 5, dans laquelle la valeur du diamètre de particule à 50 % dans la distribution cumulée (d50) de mannitol est entre 10 et 200 µm, de préférence de 20 à 100 µm et, de façon davantage préférée, elle est de 30 à 60 µm, mesurée par un analyseur de dimension de particule à diffraction laser Mastersizer 2000 de Malvern.

7. Formulation pharmaceutique se désintégrant par voie orale selon la revendication 1, dans laquelle la valeur du diamètre de particule à 50 % dans la distribution cumulée (d50) de crospovidone est entre 5 et 30 µm, de préférence de 7 à 20 µm, mesurée par un analyseur de dimension de particule à diffraction laser Mastersizer 2000 de Malvern.

8. Formulation pharmaceutique se désintégrant par voie orale selon la revendication 2, dans laquelle le lubrifiant est le stéarylfumarate de sodium.

9. Formulation pharmaceutique à désintégration orale selon l'une quelconque des revendications précédentes, dans laquelle le rapport de crospovidone au stéarylfumarate de sodium se situe dans la plage de 1 à 100 (p/p), de préférence de 3 à 25 (p/p) et de façon davantage préférée de 5 à 20 (p/p).
